# EUROPEAN PATENT SPECIFICATION

(11) **EP 2 666 403 B1**
(45) Date of publication and mention of the grant of the patent: **21.06.2017**
(21) Application number: 13166229.8
(22) Date of filing: 02.05.2013
(51) Int. Cl.: A61B 1/06

(54) **Endoscope system and processor device thereof**
Endoskopsystem, Prozessorvorrichtung damit
Système d'endoscope, dispositif de traitement de celui-ci

(30) Priority: 23.05.2012 JP 2012117725
(43) Date of publication of application: 27.11.2013
(73) Proprietor: Fujifilm Corporation, Minato-ku Tokyo (JP)
(72) Inventor: Yamaguchi, Hiroshi, Kanagawa (JP)
(74) Representative: Klunker . Schmitt-Nilson . Hirsch

(56) References cited:
- EP-A2- 2 106 736
- WO-A1-2013/049264
- US-A- 5 515 449
- US-A1- 2010 266 202

## Description

### BACKGROUND OF THE INVENTION

### 1. Field of the Invention

The present invention relates to an endoscope system and a processor device thereof, and an image producing method.

### 2. Description Related to the Prior Art

In a medical field, an endoscope system is widely used for examination and diagnosis. The endoscope system is constituted of an electronic endoscope, a light source device, and a processor device. The electronic endoscope has an elongated flexible insert section to be introduced into a patient's body and an imaging unit provided at a distal end of the insert section. The light source device supplies illumination light to the electronic endoscope. The processor device applies image processing to an image signal obtained by the electronic endoscope, and displays an endoscopic image on a monitor. In recent years, there is known a type of endoscope system that performs normal observation for imaging an internal body portion under irradiation with white light (normal light) and special observation for imaging the body portion under irradiation with specific narrow band light (special light).

As the special observation, vessel pattern observation is known in which a blood vessel in a specific depth is emphasized by applying the special light that has a wavelength having a high light absorption coefficient of hemoglobin. The vessel pattern observation allows finding out a cancer-suspected lesion according to the degree of agreement between an obtained vessel pattern and a pattern specific to cancer. The vessel pattern observation also allows diagnosing the penetration of cancer in a depth direction of living body tissue, i.e. the stage of the cancer.

In the vessel pattern observation, conventionally, the narrow band light for use as the special light is generally obtained by narrowing down broad band light such as xenon light by a band-pass filter. However, as described in European Patent Publication No. 2452610, an endoscope system using a plurality of laser beams that are emitted from a plurality of laser sources and have different wavelengths has been developed in recent years. In this endoscope system, mixed light of a first laser beam having a wavelength of 405 nm, a second laser beam having a wavelength of 445 nm, and green to red fluorescence emitted from a phosphor excited by the second laser beam is used as illumination light. The light intensity ratio between the first and second laser beams is arbitrary changeable to emphasize the blood vessel in a desired depth. Moreover, since the fluorescence having a broad wavelength band is applied in addition to the first and second laser beams, the vessel pattern observation can be performed in an extremely bright state, as compared with the case of applying only the narrow band light.

In this endoscope system, however, the vessel pattern is seen very differently in accordance with difference in an oxygen saturation level of blood, even if the light intensities of the first and second laser beams are set at regulated values. For example, when the first laser beam has a high intensity and the second laser beam has a low intensity, a vessel pattern image is more susceptible to the first laser beam. At the wavelength 405 nm of the first laser beam, a light absorption coefficient of oxyhemoglobin is higher than that of deoxyhemoglobin. Thus, the higher the oxygen saturation level of blood, the more darkly the vessel pattern is seen. The lower the oxygen saturation level of blood, the more brightly the vessel pattern is seen. On the other hand, when the first laser beam has a low intensity and the second laser beam has a high intensity, the vessel pattern image is more susceptible to the second laser beam having a wavelength of 445 nm at which the light absorption coefficient of oxyhemoglobin is lower than that of deoxyhemoglobin. Thus, the higher the oxygen saturation level of blood, the more brightly the vessel pattern is seen. The lower the oxygen saturation level of blood, the more darkly the vessel pattern is seen. The difference in vision of the vessel pattern may impair the reliability of diagnosis of the cancer and its stage. In other words, robustness, which is the ability to make the vision of the vessel pattern stable against the effect of disturbance such as noise, cannot be maintained.

In this context endoscope systems of interest are disclosed in US 2010/0266202 A1 and US-A-5 515 449.

### SUMMARY OF THE INVENTION

An object of the present invention is to provide an endoscope system and a processor device thereof and an image producing method that can produce invariant vision of a vessel pattern, no matter if an oxygen saturation level of blood changes.

To achieve the above and other objects, an endoscope system according to claim 1 is disclosed.

The contrast adjusting section preferably includes at least one of a light intensity ratio fine-adjusting section for finely adjusting a light intensity ratio between the first and second illumination light, a color tone adjusting section for adjusting a color tone of the vessel pattern image, and a gradation converting section for applying gradation conversion processing to the vessel pattern image.

The light intensity ratio fine-adjusting section may finely adjust the light intensity ratio between the first and second illumination light. After that, the gradation converting section may apply the gradation conversion processing to the vessel pattern image captured under irradiation with the first and second illumination light having finely adjusted intensity.

The contrast adjusting section adjusts change of the vessel contrast caused by change of an oxygen saturation level of blood in the vessel pattern image.

The first and second illumination light is applied simultaneously or sequentially to the internal body portion.

The endoscope system is preferably switchable between a vessel pattern imaging mode in which the vessel pattern image is displayed on a monitor and an oxygen saturation imaging mode in which an oxygen saturation image is displayed on the monitor. The oxygen saturation level of blood is imaged in the oxygen saturation image based on the image signal.

In the oxygen saturation imaging mode, the light intensity of the first or second illumination light is preferably increased in order to increase difference in brightness between a hyperoxic region having the high oxygen saturation level and a hypoxic region having the low oxygen saturation level.

It is preferable that D1≅D2 holds true, wherein D1 represents an absolute value of difference in a light absorption coefficient between the oxyhemoglobin and the deoxyhemoglobin at the first wavelength, and D2 represents an absolute value of difference in the light absorption coefficient between the oxyhemoglobin and the deoxyhemoglobin at the second wavelength.

The endoscope system may further include a third semiconductor light source for emitting third illumination light having a third wavelength to the internal body portion. The third wavelength is different from the first and second wavelengths. It is preferable D1≅D2+D3 holds true, wherein D3 represents an absolute value of difference in the light absorption coefficient between the oxyhemoglobin and the deoxyhemoglobin at the third wavelength.

A processor device of an endoscope system according to claim 13 is also disclosed.

According to the present invention, the contrast adjusting section adjusts the vessel contrast, being the contrast between the vessel area and the other area in the vessel pattern image. Thus, even if the oxygen saturation level of blood changes, the vision of the vessel pattern does not change. In other words, it is possible to maintain the robustness in the vision of the blood vessel.

### BRIEF DESCRIPTION OF THE DRAWINGS

For more complete understanding of the present invention, and the advantage thereof, reference is now made to the subsequent descriptions taken in conjunction with the accompanying drawings, in which:
Fig. 1 is a schematic view of an endoscope system;
Fig. 2 is a block diagram of the endoscope system;
Fig. 3 is a graph showing an emission spectrum of illumination light in a normal mode;
Fig. 4 is a graph showing a light absorption coefficient of oxyhemoglobin HbO2 and deoxyhemoglobin Hb;
Fig. 5A is an explanatory view of change in vessel contrast according to variation in an oxygen saturation level (StO2) in a first vessel pattern imaging mode;
Fig. 5B is an explanatory view of the vessel contrast when activating automatic exposure control;
Fig. 6A is a graph showing an emission spectrum of illumination light in a second vessel pattern imaging mode;
Fig. 6B is an explanatory view of change in the vessel contrast according to variation in the oxygen saturation level (StO2) in the second vessel pattern imaging mode;
Fig. 7A is a graph showing an emission spectrum of illumination light in a third vessel pattern imaging mode;
Fig. 7B is an explanatory view of change in the vessel contrast according to variation in the oxygen saturation level (StO2) in the third vessel pattern imaging mode;
Fig. 8A is a graph showing an emission spectrum of illumination light in an oxygen saturation imaging mode;
Fig. 8B is an explanatory view of change in the vessel contrast according to variation in the oxygen saturation level (StO2) in the oxygen saturation imaging mode;
Fig. 9 is a graph showing light transmission properties of B, G, and R color filters of a CCD;
Fig. 10A is an explanatory view of imaging control of the CCD in the normal mode;
Fig. 10B is an explanatory view of imaging control of the CCD in the first to third vessel pattern imaging modes and the oxygen saturation imaging mode;
Fig. 11 is a block diagram of a special image generator;
Fig. 12 is an explanatory view of the vessel contrast;
Fig. 13A is an explanatory view of a standard value of the vessel contrast in the second vessel pattern imaging mode;
Fig. 13B is an explanatory view of the standard value of the vessel contrast in the third vessel pattern imaging mode;
Fig. 14 is an explanatory view about fine adjustment of a light intensity ratio;
Fig. 15 is an explanatory view about gradation conversion processing;
Fig. 16 is a flowchart of the second vessel pattern imaging mode; and
Fig. 17 is a graph for explaining how to select illumination light according to another embodiment.

### DESCRIPTION OF THE PREFERRED EMBODIMENTS

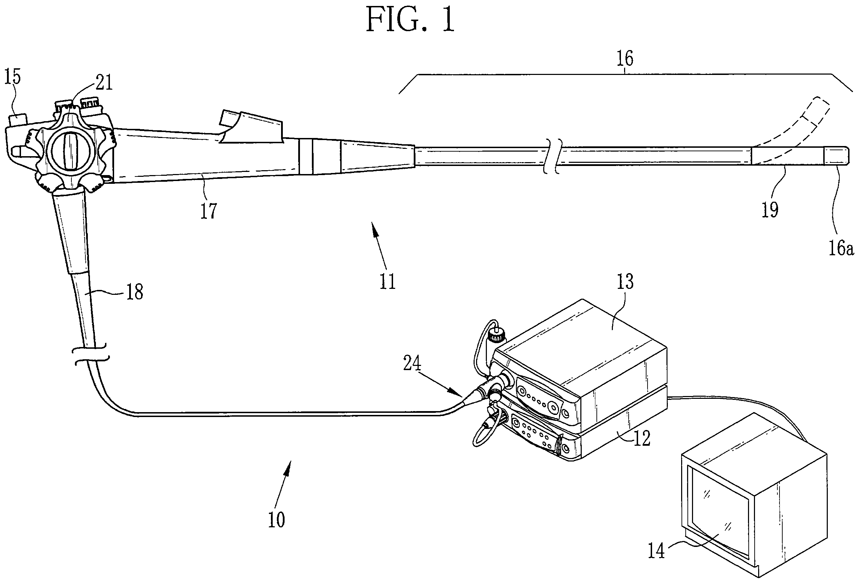

As shown in Fig. 1, an endoscope system 10 is constituted of an electronic endoscope 11, a processor device 12, a light source device 13, and a monitor 14. The electronic endoscope 11 images the interior of a human body. The processor device 12 produces an endoscopic image from a signal obtained by the electronic endoscope 11. The light source device 13 supplies illumination light to the electronic endoscope 11 to illuminate an internal body portion to be imaged. The endoscopic image is displayed on the monitor 14.

The electronic endoscope 11 is provided with a flexible insert section 16 to be introduced into a human body cavity, a control handle unit 17 provided at a proximal end of the insert section 16, and a universal cord 18 for connecting the control handle unit 17 to the processor device 12 and the light source device 13. The insert section 16 is formed with a steering assembly 19 at its distal end. The steering assembly 19 is made of a plurality of joint pieces and flexibly bent in an arbitrary direction by a turn of an angle knob 21 provided on the control handle unit 17. The steering assembly 19 is provided with a head assembly 16a at its distal end. The head assembly 16a contains an optical system for imaging the interior of the human body. By bending the steering assembly 19, the head assembly 16a is aimed at a desired direction inside the human body.

The control handle unit 17 is provided with a mode switch 15 to change a mode of the endoscope system 10. The endoscope system 10 has five modes, that is, a normal mode for observing the body portion under irradiation with broad band white light, a first vessel pattern imaging mode for observing the body portion with constant vessel contrast (contrast between a blood vessel and a mucosa) irrespective of variation in an oxygen saturation level of blood, a second vessel pattern imaging mode for displaying a vessel pattern with artificial colors, and a third vessel pattern imaging mode for enhancing the vessel pattern in a bright state, and an oxygen saturation imaging mode for observing an oxygen saturation level of blood.

The universal cord 18 has a multi connector 24, which includes a communication connector and a light source connector, at its distal end. The electronic endoscope 11 is detachably connected to the processor device 12 and the light source device 13 through this multi connector 24.

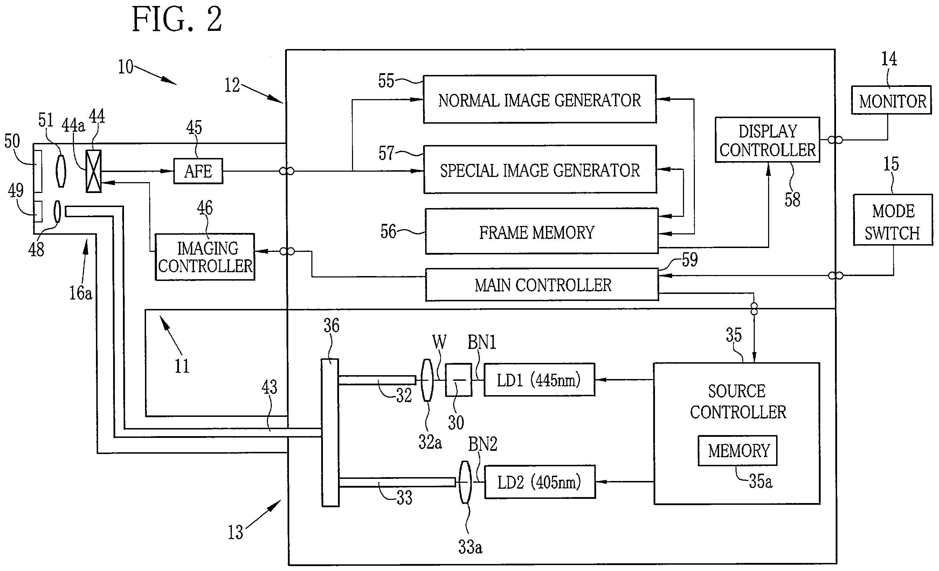

As shown in Fig. 2, the light source device 13 includes a first semiconductor light source LD1, a second semiconductor light source LD2, a phosphor 30, a first optical fiber 32, a second optical fiber 33, and a source controller 35 for controlling operation of the first and second semiconductor light sources LD1 and LD2. The first and second semiconductor light sources LD1 and LD2 are laser light sources made of a laser diode or the like. The first semiconductor light source LD1 emits a first blue laser beam BN1 having a wavelength of 445 nm ± 10 nm. The second semiconductor light source LD2 emits a second blue laser beam BN2 having a wavelength of 405 nm ± 10 nm. The first laser beam BN1 is incident upon the phosphor 30 disposed between the first semiconductor light source LD1 and the first optical fiber 32. The second laser beam BN2 enters the second optical fiber 33 through a condenser lens 33a. Note that, light emitting diodes (LEDs) may be used as the first and second semiconductor light sources LD1 and LD2.

The phosphor 30 contains a plurality of types of fluorescent substances (for example, YAG-based fluorescent substance or BAM (BaMgAl₁₀O₁₇)-based fluorescent substance). The phosphor 30 absorbs most of the first laser beam BN1 emitted from the first semiconductor light source LD1, and emits green to red fluorescence FL having a wavelength of 460 to 700 nm. The remaining first laser beam BN1 passes through the phosphor 30. Therefore, when the first laser beam BN1 is incident upon the phosphor 30, white light (pseudo white light) W is produced by mixing the fluorescence FL and the first laser beam BN1 passed through the phosphor 30. The white light W enters the first optical fiber 32 through a condenser lens 32a.

The first and second optical fibers 32 and 33 are connected to a light guide 43 of the electronic endoscope 11 via a coupler 36. Thus, the white light W transmitted through the first optical fiber 32 and the second laser beam BN2 transmitted through the second optical fiber 33 selectively or simultaneously enter the light guide 43.

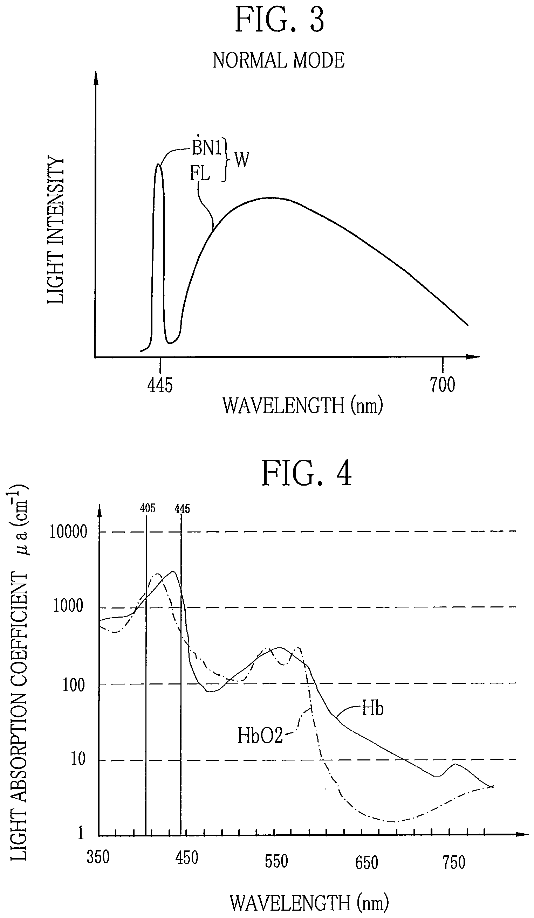

The source controller 35 is connected to a main controller 59 of the processor device 12. The source controller 35 controls the power on and off of the first and second semiconductor light sources LD1 and LD2 and the light intensity of the first and second laser beams BN1 and BN2 in accordance with the selected mode. In the normal mode, only the first semiconductor light source LD1 is turned on. Thus, the white light W having an emission spectrum shown in Fig. 3 is applied to the body portion.

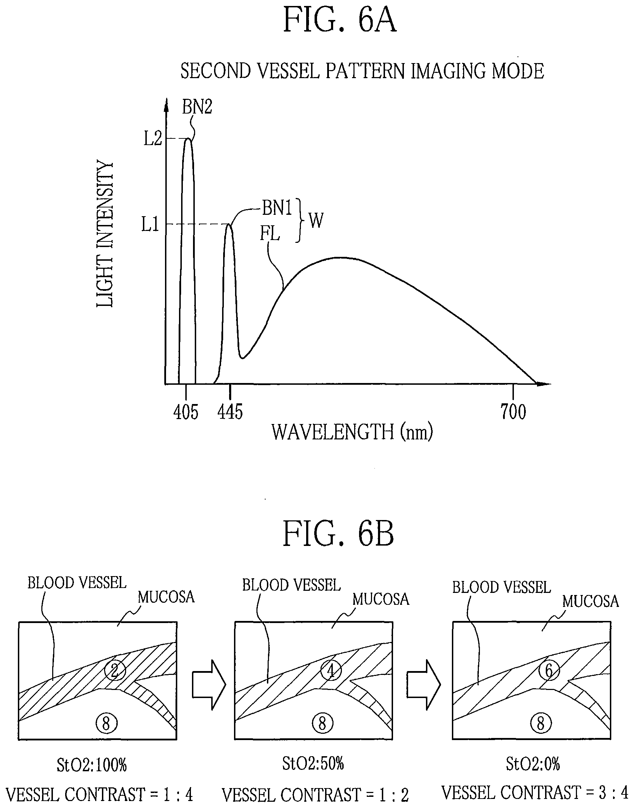

In the first to third vessel pattern imaging modes and the oxygen saturation imaging mode, both the first and second semiconductor light sources LD1 and LD2 are turned on. Thus, as shown in Figs. 6A, 7A, and 8A, the illumination light including the white light W (first laser beam BN1 + fluorescence FL) and the second laser beam BN2 is applied to the body portion. The light intensity ratio between the first and second laser beams BN1 and BN2 is predetermined differently from mode to mode, and is stored in a memory 35a of the source controller 35.

In the first vessel pattern imaging mode, the first and second laser beams BN1 and BN2 are produced at a predetermined first intensity ratio. At the first intensity ratio, the first and second laser beams BN1 and BN2 are applied at approximately the same light intensity to the body portion. As shown in Fig. 4, at the wavelength 445 nm of the first laser beam BN1, a light absorption coefficient of deoxyhemoglobin Hb is larger than that of oxyhemoglobin HbO2. Note that, an isosbestic wavelength (around 420 nm) at which deoxyhemoglobin Hb and oxyhemoglobin HbO2 have the same light absorption coefficient exists between wavelengths of 405 nm and 445 nm.

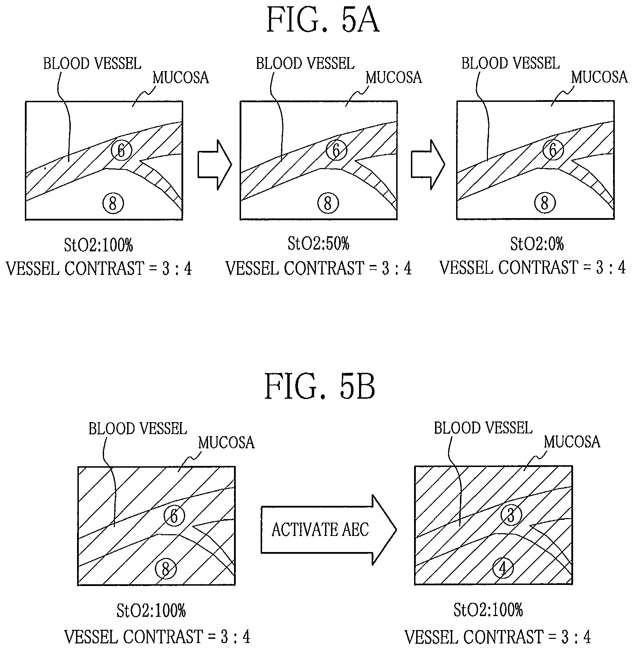

In the first vessel pattern imaging mode, as shown in Fig. 5A, the contrast (vessel contrast) between a blood vessel and a mucosa hardly varies in an image, even if the oxygen saturation level decreases from 100%, to 50%, and to 0%. In other words, the vessel pattern is observed in the same vessel contrast no matter if the oxygen saturation level StO2 varies. This image obtained in the first vessel pattern imaging mode is referred to as a first vessel pattern image.

The vessel contrast does not vary in the first vessel pattern image, even if an entire light amount (the total light amount of the first laser beam BN1, the second laser beam BN2, and the fluorescence FL) changes due to actuation of AE (auto exposure). For example, as shown in Fig. 5B, when the head assembly 16a of the electronic endoscope 11 gets near the body portion (near view) and a light amount taken in by an imaging section of the electronic endoscope 11 is increased, the AE is performed so as to reduce the entire light amount. The brightness of the vessel pattern becomes dark owing to the AE, but at the same time, the brightness of the mucosa becomes dark too. Thus, the vessel contrast does not change between before and after the AE. Likewise, the AE has no effect on the vessel contrast in the second and third vessel pattern imaging modes and the oxygen saturation imaging mode. In Figs. 5A and 5B, numbers enclosed by circles represent luminance values. The luminance value is measured on a scale from 1 to 10. A luminance value of "10" indicates the brightest state, and a luminance value of "1" indicates the darkest state.

In the second vessel pattern imaging mode, as shown in Fig. 6A, the first and second laser beams BN1 and BN2 are produced at a second intensity ratio. At the second intensity ratio, the light intensity L2 of the second laser beam BN2 is higher than the light intensity L1 of the first laser beam BN1. Thus, an image obtained in this mode is susceptible to the second laser beam BN2. As shown in Fig. 4, at the wavelength 405 nm of the second laser beam BN2, the light absorption coefficient of oxyhemoglobin HbO2 is larger than that of deoxyhemoglobin Hb. Accordingly, assuming that the AE does not work, as shown in Fig. 6B, the vessel pattern becomes brighter with decrease in the oxygen saturation level from 100%, to 50%, and to 0%. In other words, the vessel contrast varies in accordance with change of the oxygen saturation level. The variation of the vessel contrast is corrected by a light intensity ratio fine-adjusting section 62 or a gradation converting section 63, though details will be described later. The image obtained after the correction of the vessel contrast in the second vessel pattern imaging mode is referred to as a second vessel pattern image.

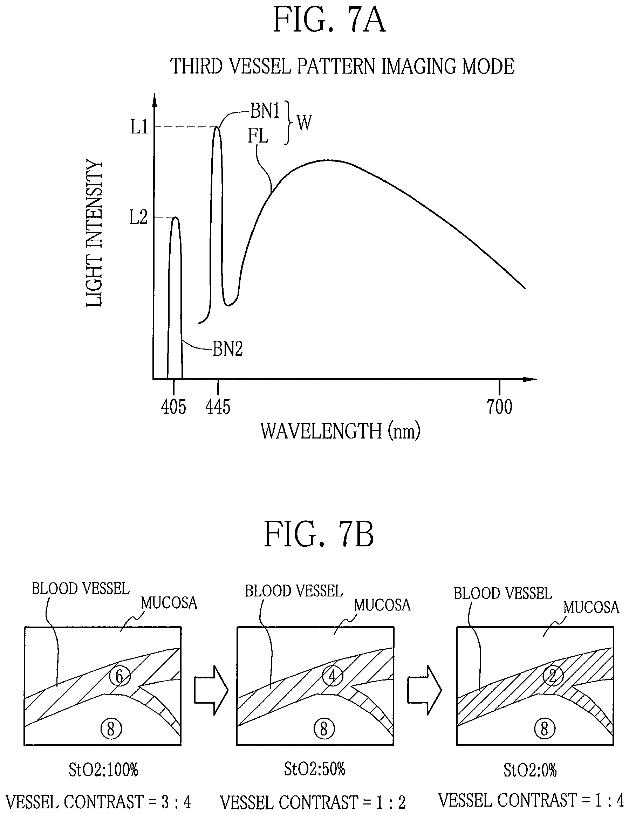

In the third vessel pattern imaging mode, as shown in Fig. 7A, the first and second laser beams BN1 and BN2 are produced at a third intensity ratio. At the third intensity ratio, the light intensity L1 of the first laser beam BN1 is higher than the light intensity L2 of the second laser beam BN2. Thus, an image obtained in this mode is susceptible to the first laser beam BN1. As shown in Fig. 4, at the wavelength 445 nm of the first laser beam BN1, the light absorption coefficient of deoxyhemoglobin Hb is larger than that of oxyhemoglobin HbO2. Accordingly, assuming that the AE does not work, as shown in Fig. 7B, the vessel pattern becomes darker with decrease in the oxygen saturation level from 100%, to 50%, and to 0%. In other words, the vessel contrast varies in accordance with change of the oxygen saturation level. The variation of the vessel contrast is corrected by the light intensity ratio fine-adjusting section 62 or the gradation converting section 63, as in the case of the second vessel pattern imaging mode. The image obtained after the correction of the vessel contrast in the third vessel pattern imaging mode is referred to as a third vessel pattern image.

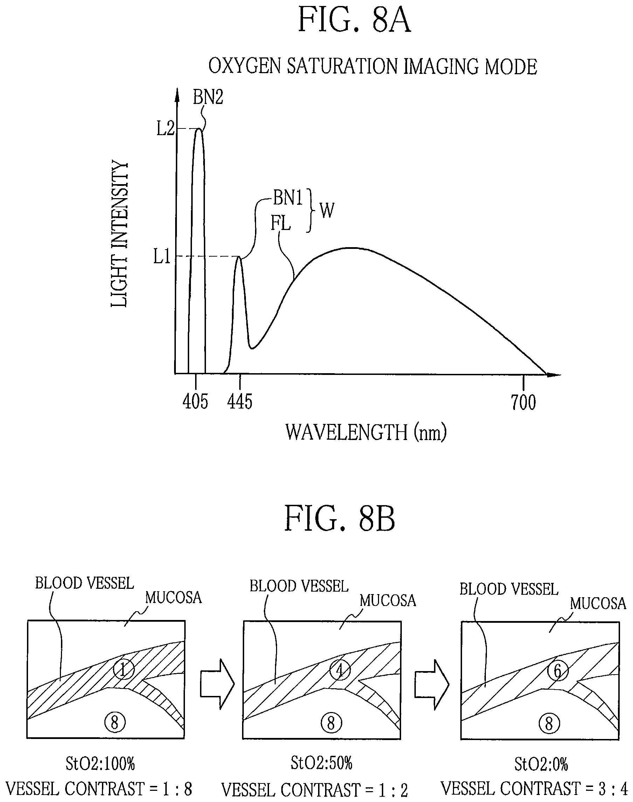

In the oxygen saturation imaging mode, as shown in Fig. 8A, the first and second laser beams BN1 and BN2 are produced at a StO2 intensity ratio. At the StO2 intensity ratio, the light intensity L2 of the second laser beam BN2 is much higher than the light intensity L1 of the first laser beam BN1. Thus, an image obtained in this mode is susceptible to the second laser beam BN2. As shown in Fig. 4, at the wavelength 405 nm of the second laser beam BN2, the light absorption coefficient of oxyhemoglobin HbO2 is larger than that of deoxyhemoglobin Hb. Accordingly, assuming that the AE does not work, as shown in Fig. 8B, the vessel pattern becomes brighter with decrease in the oxygen saturation level from 100%, to 50%, and to 0%. Taking advantage of change in the brightness of the vessel pattern, a hypoxic region and a hyperoxic region are visualized in an oxygen saturation image.

As shown in Fig. 2, the electronic endoscope 11 is provided with the light guide 43, a CCD 44, an analog frontend circuit (AFE) 45, and an imaging controller 46. The light guide 43 is made of a large diameter optical fiber, a bundle fiber, or the like. The light guide 43 is connected to the light source device 13 at its light incident end, and is aimed at a lighting lens 48 at its light exit end. The illumination light transmitted through the light guide 43 is applied to the body portion through the lighting lens 48 and a lighting window 49.

The light reflected from the body portion is incident upon an imaging surface 44a of the CCD 44 through an imaging window 50 and an image-forming lens 51. The CCD 44 photoelectrically converts the light received by the imaging surface 44a, and accumulates signal charge. The accumulated signal charge is read out as an imaging signal, and is transmitted to the AFE 45.

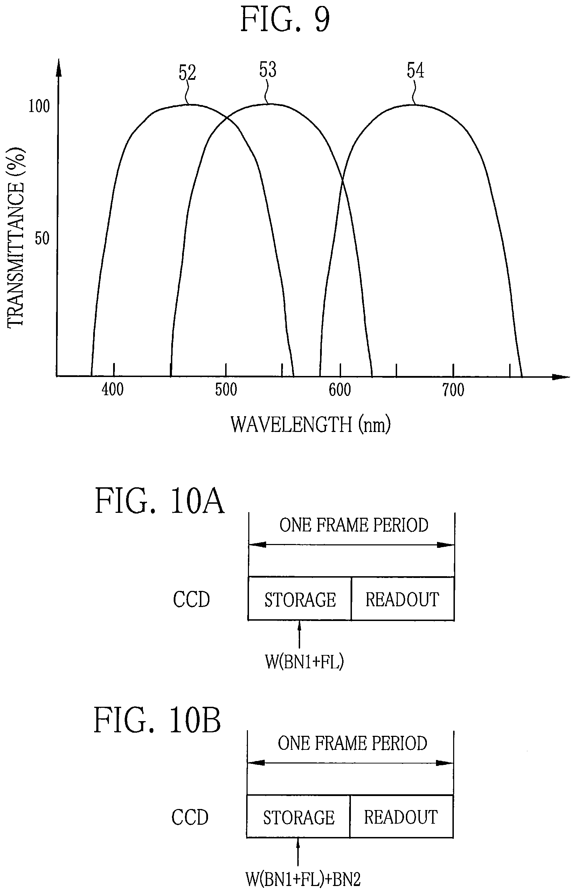

The CCD 44 is a color CCD image sensor. The imaging surface 44a has an arrangement of three-color pixels, i.e. B color pixels having a blue (B) color filter, G color pixels having a green (G) color filter, and R color pixels having a red (R) color filter. The B, G, and R color filters have spectral transmittance represented by curves 52, 53, and 54 of Fig. 9, respectively. Thus, the B color pixel receives light having a wavelength of 380 to 560 nm. The G color pixel receives light having a wavelength of 450 to 630 nm. The R color pixel receives light having a wavelength of 580 to 760 nm. Instead of the CCD image sensor, a CMOS image sensor having RGB color filters may be used. Another CCD image sensor or CMOS image sensor that has not the RGB color filters but CMY color filters, being complimentary color filters, may be used instead.

The AFE 45 is composed of a correlated double sampling circuit (CDS), an automatic gain controller (AGC), and an analog-to-digital converter (A/D), none of which is illustrated though. The CDS applies correlated double sampling processing to an image signal outputted from the CCD 44, in order to remove noise caused by operation of the CCD 44. The AGC amplifies the image signal after the noise reduction by the CDS. The A/D converts the amplified image signal into a digital image signal of a predetermined bit number, and inputs the digital image signal to the processor device 12.

The imaging controller 46 is connected to the main controller 59 of the processor device 12. The imaging controller 46 issues a drive signal to the CCD 44 under control of the main controller 59. In response to the drive signal, the CCD 44 outputs the image signal to the AFE 45 at a predetermined frame rate.

In the normal mode, as shown in Fig. 10A, the signal charge is stored and read out within one frame period under irradiation with the white light W (first laser beam BN1 + fluorescence FL). The storage and readout is repeatedly performed as long as the endoscope system 2 is in the normal mode. Thus, a blue signal Bc, a green signal Gc, and a red signal Rc are read out as the image signals from the B, G, and R pixels of the CCD 44, respectively.

In the first to third vessel pattern imaging modes and the oxygen saturation imaging mode, as shown in Fig. 10B, the storage and readout of the signal charge is performed under irradiation with the white light W (first laser beam BN1 + fluorescence FL) and the second laser beam BN2. Thus, a blue signal Bs, a green signal Gs, and a red signal Rs are read out as the image signals from the B, G, and R pixels of the CCD 44, respectively.

As shown in Fig. 2, the processor device 12 includes a normal image generator 55, a frame memory 56, a special image generator 57, and a display controller 58, which are controlled by the main controller 59. The normal image generator 55 produces a normal image from the image signals Bc, Gc, and Rc obtained in the normal mode. B, G, and R images of the normal image are assigned to B, G, and R channels of the monitor 14, respectively. The normal image is temporarily stored to the frame memory 56 before being displayed on the monitor 14.

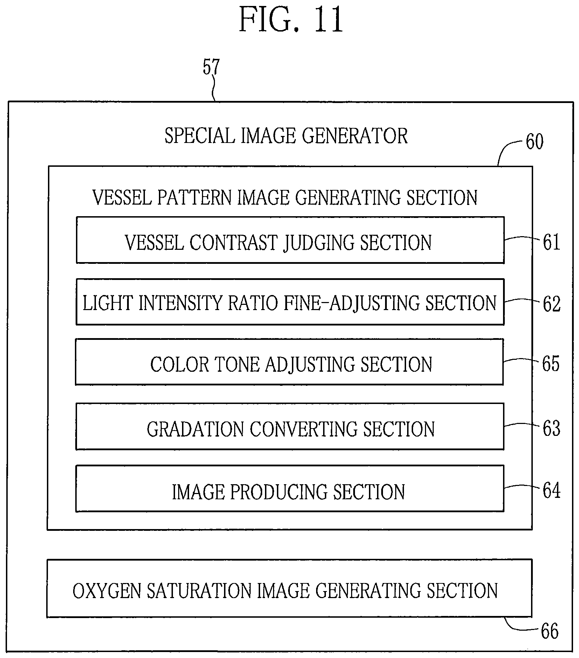

As shown in Fig. 11, the special image generator 57 is constituted of a vessel pattern image generating section 60 and an oxygen saturation image generating section 66. The vessel pattern image generating section 60 produces the first to third vessel pattern images from the image signals Bs, Gs, and Rs obtained in the first to third vessel pattern imaging modes. The oxygen saturation image generating section 66 produces the oxygen saturation image from the image signals Bs, Gs, and Rs obtained in the oxygen saturation imaging mode. The vessel pattern image generating section 60 includes a vessel contrast judging section 61, a light intensity ratio fine-adjusting section 62, a color tone adjusting section 65, a gradation converting section 63, and an image producing section 64. The vessel contrast judging section 61 judges whether or not the vessel contrast coincides with a standard value based on the image signals Bs, Gs, and Rs obtained in the second or third vessel pattern imaging mode. When the vessel contrast does not coincide with the standard value, the light intensity ratio fine-adjusting section 62 finely adjusts the second or third intensity ratio. Then imaging is performed again to obtain the second or third vessel pattern image having the standard vessel contrast.

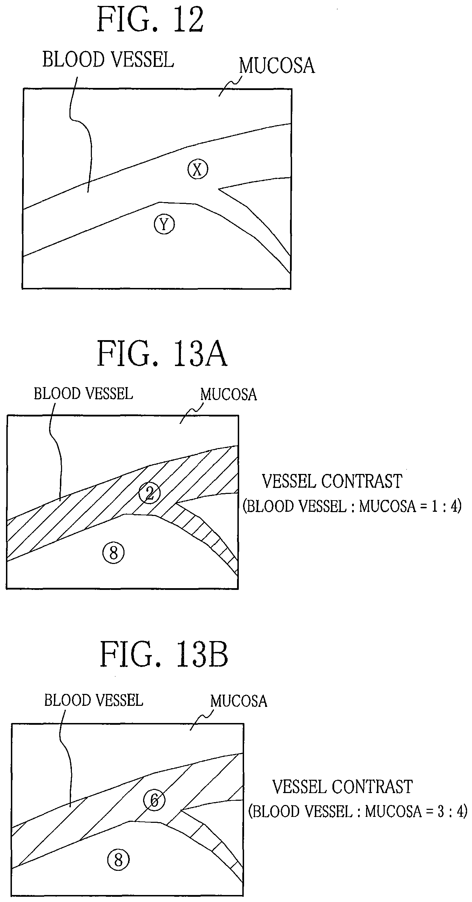

To be more specific, the vessel contrast judging section 61 produces a B/G ratio image, which represents the light intensity ratio between the blue signal Bs and the green signal Gs obtained in the second or third vessel pattern imaging mode. In the B/G ratio image, pixels having the B/G ratio that is within a predetermined range constitute a mucosa area, while the other pixels having the B/G ratio that is out of the predetermined range constitute a vessel area. Then, as shown in Fig. 12, the ratio (X:Y) between a light intensity value X of the vessel area and a light intensity value Y of the mucosa area is calculated as the vessel contrast. Whether or not the calculated vessel contrast coincides with the standard value is judged.

The vessel contrast in a state where the oxygen saturation level is 100% is used as the standard value. In the second vessel pattern imaging mode, the vessel pattern becomes the darkest when the oxygen saturation level is 100%. The vessel pattern becomes brighter with decrease in the oxygen saturation level. As shown in Fig. 13A, when the oxygen saturation level is 100%, if the light intensity value of the vessel area is "2" and the light intensity value of the mucosa area is "8", the standard value of the vessel contrast is "1:4".

In the third vessel pattern imaging mode, on the other hand, the vessel pattern becomes the brightest when the oxygen saturation level is 100%. The vessel pattern becomes darker with decrease in the oxygen saturation level. As shown in Fig. 13B, when the oxygen saturation level is 100%, if the light intensity value of the vessel area is "6" and the light intensity value of the mucosa area is "8", the standard value of the vessel contrast is "3: 4". Note that, when the body portion to be imaged includes arteries, the vessel contrast in a state where the oxygen saturation level is 100% is preferably used as the standard value. When the body portion includes many veins, the vessel contrast in a state where the oxygen saturation level is 70% is preferably used as the standard value.

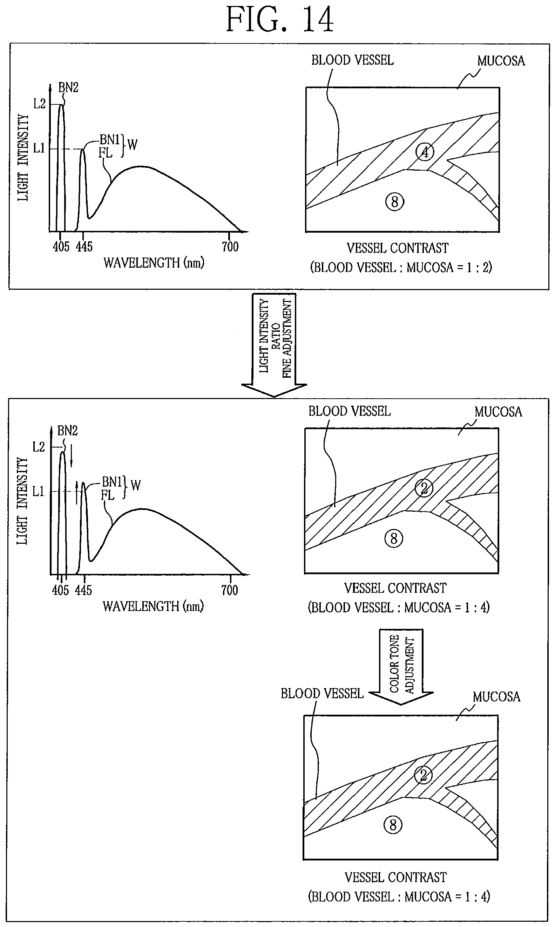

When the vessel contrast does not coincide with the standard value, the light intensity ratio fine-adjusting section 62 corrects the vessel contrast by fine adjustment of the second or third intensity ratio. In the second vessel pattern imaging mode, as shown in Fig. 14, the light intensity of the second laser beam BN2 having a wavelength of 405 nm is higher than that of the first laser beam BN1 having a wavelength of 445 nm. When the vessel pattern becomes bright due to decrease in the oxygen saturation level, the vessel contrast does not coincide with the standard value.

Therefore, the light intensity ratio fine-adjusting section 62 drives the source controller 35 of the light source device 13 so as to decrease the light intensity of the second laser beam BN2 and increase the light intensity of the first laser beam BN1. Thus, the second intensity ratio is finely adjusted, so the vessel pattern becomes dark just as in the case of the oxygen saturation level of 100%. In a case where a color tone of the mucosa is changed owing to the fine adjustment of the second intensity ratio, the color tone adjusting section 65 adjusts the color tone of the mucosa area. In the third vessel pattern imaging mode, the fine adjustment of the third intensity ratio is performed in a like manner.

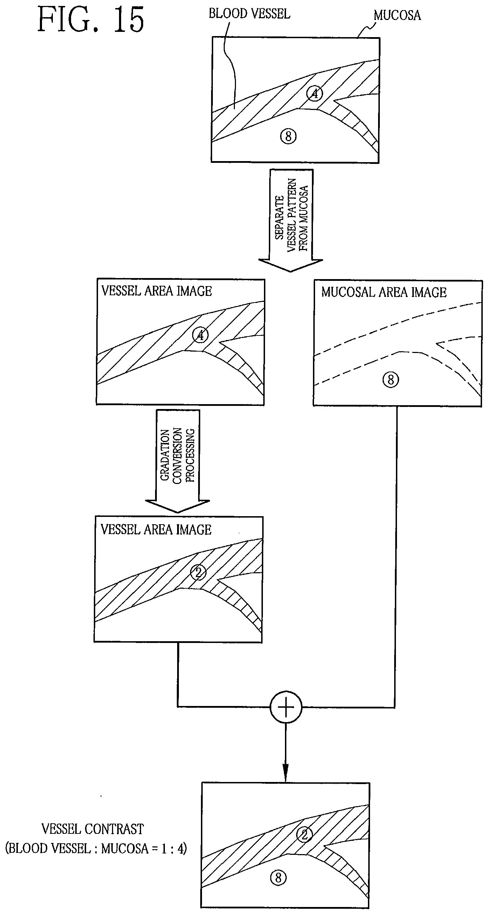

When the vessel contrast does not coincide with the standard value, the gradation converting section 63 may correct the vessel contrast by converting a gradation of the blood vessel, instead of or in addition to the correction of the vessel contrast by the light intensity ratio fine-adjusting section 62. In the second vessel pattern imaging mode, as described above, when the vessel pattern becomes bright due to decrease in the oxygen saturation level, the vessel contrast does not coincide with the standard value. In this case, as shown in Fig. 15, the gradation converting section 63 produces a vessel area image, which shows only the vessel area, and a mucosa area image, which shows an area excluding the vessel area and including the mucosa area and the like, from the image signals Bs, Gs, and Rs obtained in the second vessel pattern imaging mode. The vessel area image is produced by extracting the vessel area in which the signal ratio (B/G ratio) between the blue signal Bs and the green signal Gs is out of the predetermined range. The mucosa area image is produced by extracting the mucosa area in which the B/G ratio is within the predetermined range.

Then, the gradation converting section 63 applies gradation conversion processing to the vessel area image so as to convert a luminance value of "4" of the vessel area into a luminance value of "2", which is a luminance value of the vessel area in a state where the oxygen saturation level is 100%. The vessel area image after being subjected to the gradation conversion processing is merged with the mucosa area image, to obtain an image that has the vessel contrast coinciding with the standard value. In the third vessel pattern imaging mode, the gradation conversion processing is carried out in a like manner.

In the first vessel pattern imaging mode, the image producing section 64 produces the first vessel pattern image from the image signals Bs, Gs, and Rs obtained by the electronic endoscope 11. B, G, and R images of the first vessel pattern image are assigned to the B, G, and R channels of the monitor 14, respectively. In the first vessel pattern image, as shown in Fig. 5A, the vessel contrast is invariant irrespective of change of the oxygen saturation level, so the vessel pattern is seen in the same manner. The first vessel pattern image is temporarily stored to the frame memory 56 before being displayed on the monitor 14. The same goes for the second and third vessel pattern images as described below.

In the second vessel pattern imaging mode, the image producing section 64 produces the second vessel pattern image from the image signals Bs, Gs, and Rs obtained under irradiation with the illumination light having the fine-adjusted second intensity ratio or the blue signal Bs and the green signal Gs after being subjected to the gradation conversion processing. A B image of the second vessel pattern image is assigned to the B and G channels of the monitor 14, while a G image of the second vessel pattern image is assigned to the R channel of the monitor 14. Thus, the entire body portion including the vessel pattern is colored artificially in the second vessel pattern image.

In the third vessel pattern imaging mode, the image producing section 64 produces the third vessel pattern image from the image signals Bs, Gs, and Rs obtained under irradiation with the illumination light having the fine-adjusted third intensity ratio or the image signals Bs, Gs, and Rs after being subjected to the gradation conversion processing. B, G, and R images of the third vessel pattern image are assigned to the B, G, and R channels of the monitor 14, respectively. Thus, in the third vessel pattern image, the vessel pattern is emphasized on living body tissue that is displayed with color having brightness of a certain value or more.

The oxygen saturation image generating section 66 has a correlation memory (not shown) that stores in advance the correlation between the oxygen saturation level and the image signals Bs, Gs, and Rs. The oxygen saturation level is obtained from this correlation by looking up an oxygen saturation value based on the image signals Bs, Gs, and Rs obtained in the oxygen saturation imaging mode. Based on the obtained oxygen saturation level, the oxygen saturation image is produced. Note that, in the oxygen saturation image, the entire image may be assigned with artificial colors in accordance with the oxygen saturation level. Alternatively, only the hypoxic region having a low oxygen saturation value may be colored artificially, while the remains of the image may be colored just as with the normal image.

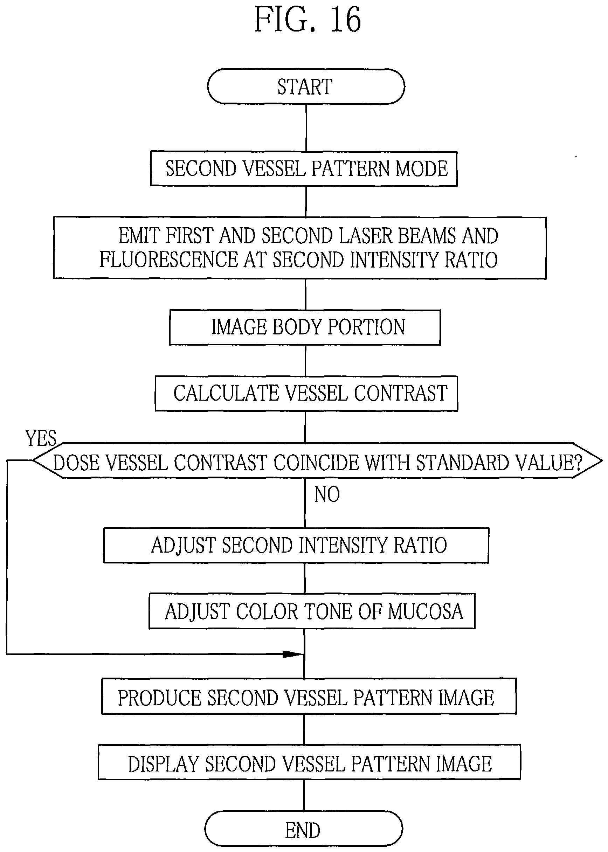

Next, the operation of the second vessel pattern imaging mode will be described with referring to a flowchart of Fig. 16. The operation of the third vessel pattern imaging mode is the same as that of the second vessel pattern imaging mode, so the description thereof will be omitted. In the second vessel pattern imaging mode, the first and second laser beams BN1 and BN2 and the fluorescence FL are applied to the body portion at the second intensity ratio at which the intensity of the second laser beam BN2 is higher than that of the first laser beam BN1. The color CCD 44 takes an image of the body portion under irradiation with the first and second laser beams BN1 and BN2 and the fluorescence FL, to obtain the image signals Bs, Gs, and Rs.

The vessel contrast being the contrast between the blood vessel and the mucosa is calculated from the image signals Bs, Gs, and Rs. When the vessel contrast coincides with the predetermined standard value, the second vessel pattern image is produced from the image signals Bs, Gs, and Rs. When the vessel contrast dose not coincide with the standard value, on the other hand, the second intensity ratio is finely adjusted so that the vessel contrast is made coincide with the standard value. In the fine adjustment of the second intensity ratio, the light intensity of the second laser beam BN2 is decreased and the light intensity of the first laser beam BN1 is increased, while keeping the entire light intensity (the total light intensity of the first laser beam BN1, the second laser beam BN2, and the fluorescence FL) constant. In a case where the fine adjustment of the second intensity ratio brings about change in a color tone of the mucosa area, color tone adjustment is performed so as to convert the color tone of the mucosa area back into a state before the fine adjustment. After that, the second vessel pattern image is produced from the image signals Bs, Gs, and Rs obtained after the fine adjustment of the second intensity ratio. The produced second vessel pattern image is displayed on the monitor 14.

In the above embodiments, in the first vessel pattern imaging mode, the light intensity ratio between the first and second blue laser beams BN1 and BN2, which have different wavelengths between which the isosbestic wavelength exists, is set at a predetermined value, for the purpose of keeping the vessel contrast constant no matter if the oxygen saturation level StO2 is changed. However, to keep the vessel contrast constant, narrow band light may be used that has two wavelengths between which the isosbestic wavelength exists and between which an absolute value of the difference in the light absorption coefficient between oxyhemoglobin and deoxyhemoglobin is the same. Alternatively, narrow band light having another single wavelength may be used, in addition to narrow band light having two wavelengths between the which isosbestic wavelength exists.

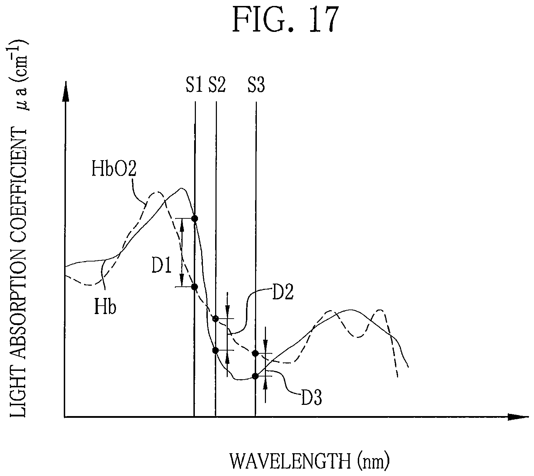

For example, as shown in Fig. 17, when two types of narrow band light having wavelengths S1 and S2 are applied simultaneously to the body portion, an absolute value D1 of the difference in the light absorption coefficient between oxyhemoglobin and deoxyhemoglobin at the wavelength S1 is larger than an absolute value D2 of the difference in the light absorption coefficient between oxyhemoglobin and deoxyhemoglobin at the wavelength S2. Accordingly, in order to keep the vessel contrast constant irrespective of change of the oxygen saturation level StO2, the light intensity ratio between the two types of narrow band light must be set appropriately. Instead of this, another type of narrow band light having a wavelength S3 is applied in addition to the two types of narrow band light. At the wavelength S3, just as with the wavelength S2, the light absorption coefficient of oxyhemoglobin is higher than that of deoxyhemoglobin. "D3" represents an absolute value of the difference in the light absorption coefficient between oxyhemoglobin and deoxyhemoglobin. If "D1≅D2+D3" holds true, the vessel contrast can be kept constant even if every type of narrow band light is applied at the same light intensity.

In the above embodiments, the first and second laser beams BN1 and BN2 are applied simultaneously. However, the first and second laser beams BN1 and BN2 may be applied sequentially, and images may be captured sequentially in synchronization with the application of the first and second laser beams BN1 and BN2. The image captured under irradiation with the first laser beam BN1 may be merged with the image captured under irradiation with the second laser beam BN2, and the merged image may be subjected to image processing to make the vessel contrast constant.

In the above embodiments, after the fine adjustment of the second intensity ratio, the color tone of the mucosa is adjusted to make the vessel contrast constant. Instead of this, the gradation conversion processing may be applied to the mucosa area in the image after the fine adjustment of the second intensity ratio.

The above embodiments describe the endoscope system in which the vessel contrast is adjusted in the image obtained by the electronic endoscope. However, the present invention is applicable to another type of endoscope system such as a fiber scope by which the internal body portion is seen directly under irradiation with the illumination light. In this case, the endoscope system emits a plurality of types of illumination light the light intensity of which is independently adjustable. The light intensity ratio among the plurality of types of illumination light is finely adjusted, in order to produce vision of object tissue having the invariant vessel contrast.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope system (10) comprising:
a first semiconductor light source for emitting first illumination light having a first wavelength;
a second semiconductor light source for emitting second illumination light having a second wavelength, an isosbestic wavelength of oxyhemoglobin and deoxyhemoglobin existing between said first and second wavelengths;
an imaging device (11) for imaging an internal body portion irradiated with said first or second illumination light;
a vessel pattern image generating section (60) for producing a vessel pattern image based on an image signal obtained by said imaging device (11) including
a vessel contrast judging section (61) for judging whether or not a vessel contrast between a vessel area and an area other than said vessel area in said vessel pattern image coincides with a standard value; and
a contrast adjusting section (62, 63, 65) for adjusting said vessel contrast in the case where said vessel contrast does not coincide with said standard value,
**characterized in that** the endoscope system further comprises an oxygen saturation image generating section for producing an oxygen saturation image based on an image signal obtained by said imaging device, and said contrast adjusting section (62, 63, 65) is configured to adjust change of said vessel contrast caused by change of an oxygen saturation level of blood in said vessel pattern image, and
the light intensity of said first illumination light is higher than the light intensity of said second illumination light, or the light intensity of said second illumination light is higher than the light intensity of said first illumination light.

2. The endoscope system (10) according to claim 1, wherein said contrast adjusting section includes a light intensity ratio fine-adjusting section (62) for finely adjusting a light intensity ratio between said first and second illumination light.

3. The endoscope system (10) according to claim 2, wherein said contrast adjusting section further includes a color tone adjusting section (65) for adjusting a color tone of said vessel pattern image.

4. The endoscope system (10) according to claim 1, wherein said contrast adjusting section includes a gradation converting section (63) for applying gradation conversion processing to said vessel pattern image.

5. The endoscope system (10) according to claim 2 or 3, wherein said contrast adjusting section further includes a gradation converting section (63) for applying gradation conversion processing to said vessel pattern image.

6. The endoscope system (10) according to claim 5, wherein said light intensity ratio fine-adjusting section (62) finely adjusts said light intensity ratio between said first and second illumination light; and
said gradation converting section (63) applies said gradation conversion processing to said vessel pattern image captured under irradiation with said first and second illumination light having finely adjusted intensity.

7. The endoscope system (10) according to one of claims 1 to 6, further comprising a source controller (35) for controlling operation of said first and second semiconductor light sources such that said first and second illumination light is applied simultaneously to said internal body portion.

8. The endoscope system (10) according to one of claims 1 to 6, further comprising a source controller (35) for controlling operation of said first and second semiconductor light sources such that said first and second illumination light is applied sequentially to said internal body portion.

9. The endoscope system (10) according to one of claims 1 to 8 being switchable between a vessel pattern imaging mode in which said vessel pattern image is displayed on a monitor and an oxygen saturation imaging mode in which an oxygen saturation image is displayed on said monitor, an oxygen saturation level of blood being imaged in said oxygen saturation image based on said image signal.

10. The endoscope system (10) according to claim 9, wherein in said oxygen saturation imaging mode, said light intensity of said first illumination light is made higher than that of said second illumination light, or said light intensity of said second illumination light is made higher than that of said first illumination light, in order to increase difference in brightness between a hyperoxic region having said high oxygen saturation level and a hypoxic region having said low oxygen saturation level.

11. The endoscope system (10) according to one of claims 1 to 10, wherein
D1≅D2 holds true, wherein
D1 represents an absolute value of difference in a light absorption coefficient between said oxyhemoglobin and said deoxyhemoglobin at said first wavelength; and
D2 represents an absolute value of difference in said light absorption coefficient between said oxyhemoglobin and said deoxyhemoglobin at said second wavelength.

12. The endoscope system (10) according to one of claims 1 to 10, further comprising:
a third semiconductor light source for emitting third illumination light having a third wavelength to said internal body portion, said third wavelength being different from said first and second wavelengths; wherein
D1≅D2+D3 holds true, wherein
D1 represents an absolute value of difference in a light absorption coefficient between said oxyhemoglobin and said deoxyhemoglobin at said first wavelength;
D2 represents an absolute value of difference in said light absorption coefficient between said oxyhemoglobin and said deoxyhemoglobin at said second wavelength; and
D3 represents an absolute value of difference in said light absorption coefficient between said oxyhemoglobin and said deoxyhemoglobin at said third wavelength.

13. A processor device (12) of an endoscope system (10) including an electronic endoscope (11) for imaging an internal body portion under irradiation with first illumination light having a first wavelength and second illumination light having a second wavelength, an isosbestic wavelength of oxyhemoglobin and deoxyhemoglobin existing between said first and second wavelengths, said processor device (12) comprising:
a vessel pattern image generating section (60) for producing a vessel pattern image based on an image signal obtained by said electronic endoscope (11) including
a vessel contrast judging section (61) for judging whether or not a vessel contrast between a vessel area and an area other than said vessel area in said vessel pattern image coincides with a standard value; and
a contrast adjusting section (62, 63, 65) for adjusting said vessel contrast in the case where said vessel contrast does not coincide with said standard value,
**characterized in that** the endoscope system further comprises an oxygen saturation image generating section for producing an oxygen saturation image based on an image signal obtained by said imaging device, and said contrast adjusting section (62, 63, 65) is configure to adjust change of said vessel contrast caused by change of an oxygen saturation level of blood in said vessel pattern image, and
the light intensity of said first illumination light is higher than the light intensity of said second illumination light, or the light intensity of said second illumination light is higher than the light intensity of said first illumination light.

## Patentansprüche

1. Endoskopsystem (10), umfassend:
eine erste Halbleiterlichtquelle zum Emittieren von erstem Beleuchtungslicht mit einer ersten Wellenlänge;
eine zweite Halbleiterlichtquelle zum Emittieren von zweitem Beleuchtungslicht mit einer zweiten Wellenlänge, wobei zwischen der ersten und der zweiten Wellenlänge eine isosbestische Wellenlänge von Oxyhämoglobin und Deoxyhämoglobin existiert;
eine Bildaufnahmeeinrichtung (11) zum Abbilden eines internen Körperteils, der mit dem ersten oder dem zweiten Beleuchtungslicht bestrahlt wird;
einen Gefäßmuster-Bilderzeugungsabschnitt (60) zum Erzeugen eines Gefäßmusterbilds basierend auf einem von der Bildaufnahmeeinrichtung (11) erhaltenen Bildsignal, enthaltend
einen Gefäß-Kontrastbeurteilungsabschnitt (61) zum Beurteilen, ob ein Gefäßkontrast zwischen einer Gefäßfläche und einer von dieser Gefäßfläche verschiedenen Fläche innerhalb des Gefäßmusterbilds mit einem Standardwert übereinstimmt oder nicht; und
einen Kontrasteinstellabschnitt (62, 63, 65) zum Einstellen des Gefäßkontrastes für den Fall, dass der Gefäßkontrast nicht mit dem Standardwert übereinstimmt,
**dadurch gekennzeichnet, dass** das Endoskop weiterhin einen Sauerstoffsättigungs-Bilderzeugungsabschnitt zum Erzeugen eines Sauerstoffsättigungsbilds basierend auf einem von der Bildaufnahmeeinrichtung erhaltenen Bildsignal aufweist, der Gefäßkontrast in einem Zustand, in welchem der Sauerstoffsättigungspegel 100% beträgt, der Standardwert ist, und dass der Kontrasteinstellabschnitt (62, 63, 65) konfiguriert ist zum Einstellen eine Änderung des Gefäßkontrasts, verursacht durch eine Änderung eines Sauerstoffsättigungswerts von Blut in dem Gefäßmusterbild, und
die Lichtintensität des ersten Beleuchtungslichts höher ist als die Lichtintensität des zweiten Beleuchtungslichts, oder die Lichtintensität des zweiten Beleuchtungslichts höher ist als die Lichtintensität des ersten Beleuchtungslichts.

2. Endoskopsystem (10) nach Anspruch 1, bei dem der Kontrasteinstellabschnitt einen Lichtintensitätsverhältnis-Feineinstellabschnitt (62) enthält zum Feineinstellen eines Lichtintensitätsverhältnisses zwischen dem ersten und dem zweiten Beleuchtungslicht.

3. Endoskopsystem (10) nach Anspruch 2, bei dem der Kontrasteinstellabschnitt weiterhin einen Farbton-Einstellabschnitt (65) zum Einstellen eines Farbtons des Gefäßmusterbilds aufweist.

4. Endoskopsytem (10) nach Anspruch 1, bei dem der Kontrasteinstellabschnitt einen Gradationswandlerabschnitt (63) zum Anwenden einer Gradationsumwandlungsverarbeitung auf das Gefäßmusterbild aufweist.

5. Endoskopsytem (10) nach Anspruch 2 oder 3, bei dem der Kontrasteinstellabschnitt weiterhin einen Gradationsumwandlungsabschnitt (63) zum Anwenden einer Gradationsumwandlungsverarbeitung auf das Gefäßmusterbild aufweist.

6. Endoskopsytem (10) nach Anspruch 5, bei dem
der Lichtintensitätsverhältnis-Feineinstellabschnitt (62) eine Feineinstellung des Lichtintensitätsverhältnisses zwischen dem ersten und dem zweiten Beleuchtungslicht vornimmt; und
der Gradationsumwandlungsabschnitt (63) die Gradationsumwandlungsverarbeitung auf das Gefäßmusterbild anwendet, welches unter Bestrahlung mit dem ersten und dem zweiten Beleuchtungslicht, deren Intensität einer Feineinstellung unterzogen wurde, aufgenommen wurde.

7. Endoskopsystem (10) nach einem der Ansprüche 1 bis 6, weiterhin umfassend eine Quellensteuerung (35) zum Steuern des Betriebs der ersten und der zweiten Halbleiterlichtquelle derart, dass das erste und das zweite Beleuchtungslicht gleichzeitig auf den internen Körperteil aufgebracht werden.

8. Endoskopsystem (10) nach einem der Ansprüche 1 bis 6, weiterhin umfassend eine Quellensteuerung (35) zum Steuern des Betriebs der ersten und der zweiten Halbleiterlichtquelle derart, dass das erste und das zweite Beleuchtungslicht sequentiell auf den internen Körperteil aufgebracht werden.

9. Endoskopsystem (10) nach einem der Ansprüche 1 bis 8, umschaltbar zwischen einem Gefäßmuster-Bildgebungsmodus, bei dem das Gefäßmusterbild auf einem Monitor angezeigt wird, und einem Sauerstoffsättigungs-Bildgebungsmodus, bei dem ein Sauerstoffsättigungsbild auf dem Monitor angezeigt wird, wobei der Sauerstoffsättigungspegel von Blut in dem Sauerstoffsättigungsbild basierend auf dem Bildsignal abgebildet wird.

10. Endoskopsystem (10) nach Anspruch 9, bei dem in dem Sauerstoffsättigungs-Bildgebungsmodus Lichtintensität des ersten Beleuchtungslichts höher gemacht wird als die des zweiten Beleuchtungslichts, oder die Lichtintensität des zweiten Beleuchtungslichts höher gemacht wird als die des ersten Beleuchtungslichts, um eine Helligkeitsdifferenz zwischen einer hyperoxischen Zone mit hoher Sauerstoffsättigung und einer hypoxischen Zone mit geringer Sauerstoffsättigung zu verstärken.

11. Endoskopsystem (10) nach einem der Ansprüche 1 bis 10, bei dem
D1 ≅ D2 gilt, wobei
D1 einen Absolutwert einer Differenz eines Lichtabsorptionskoeffizienten zwischen Oxyhämoglobin und Deoxyhämoglobin bei der ersten Wellenlänge bezeichnet; und
D2 einen Absolutwert einer Differenz des Lichtabsorptionskoeffizienten zwischen dem Oxyhämoglobin und dem Deoxyhämoglobin bei der zweiten Wellenlänge bezeichnet.

12. Endoskopsystem (10) nach einem der Ansprüche 1 bis 10, weiterhin umfassend:
eine dritte Halbleiterlichtquelle zum Emittieren von drittem Beleuchtungslicht mit einer dritten Wellenlänge auf den internen Körperteil, wobei die dritte Wellenlänge sich von der ersten und der zweiten Wellenlänge unterscheidet; wobei D1 ≅ D2 + D3 gilt, wobei
D1 einen Absolutwert einer Differenz eines Lichtabsorptionskoeffizienten zwischen Oxyhämoglobin und Deoxyhämoglobin bei der ersten Wellenlänge bezeichnet;
D2 einen Absolutwert einer Differenz des Lichtabsorptionskoeffizienten zwischen dem Oxyhämoglobin und dem Deoxyhämoglobin bei der zweiten Wellenlänge bezeichnet; und
D3 einen Absolutwert einer Differenz in dem Lichtabsorptionskoeffizienten zwischen dem Oxyhämoglobin und dem Deoxyhämoglobin bei der dritten Wellenlänge bezeichnet.

13. Prozessorgerät (12) eines Endoskopsystems (10), das ein elektronisches Endoskop (11) zur Bildgebung eines internen Körperteils bei Bestrahlung mit erstem Beleuchtungslicht einer ersten Wellenlänge und zweitem Beleuchtungslicht einer zweiten Wellenlänge enthält, wobei zwischen der ersten und der zweiten Wellenlänge eine isosbestische Wellenlänge von Oxyhämoglobin und Deoxyhämoglobin existiert, wobei das Prozessorgerät (12) aufweist:
einen Gefäßmusterbild-Erzeugungsabschnitt (60) zum Erzeugen eines Gefäßmusterbilds basierend auf einem von dem elektronischen Endoskop (11) erhaltenen Bildsignal, enthaltend
einen Gefäßkontrast-Beurteilungsabschnitt (61) zum Beurteilen, ob ein Gefäßkontrast zwischen einer Gefäßfläche und einer von de Gefäßfläche verschiedenen Fläche in dem Gefäßmusterbild mit einem Standardwert übereinstimmt; und
einen Kontrasteinstellabschnitt (62, 63, 65) zum Einstellen des Gefäßkontrasts für den Fall, dass der Gefäßkontrast nicht mit dem Standardwert übereinstimmt,
**dadurch gekennzeichnet, dass** das Endoskop weiterhin einen Sauerstoffsättigungs-Bilderzeugungsabschnitt zum Erzeugen eines Sauerstoffsättigungsbilds basierend auf einem von der Bildaufnahmeeinrichtung erhaltenen Bildsignal aufweist, der Gefäßkontrast in einem Zustand, in welchem der Sauerstoffsättigungspegel 100% beträgt, der Standardwert ist, und dass der Kontrasteinstellabschnitt (62, 63, 65) konfiguriert ist zum Einstellen eine Änderung des Gefäßkontrasts, verursacht durch eine Änderung eines Sauerstoffsättigungswerts von Blut in dem Gefäßmusterbild, und
die Lichtintensität des ersten Beleuchtungslichts höher ist als die Lichtintensität des zweiten Beleuchtungslichts, oder die Lichtintensität des zweiten Beleuchtungslichts höher ist als die Lichtintensität des ersten Beleuchtungslichts.

## Revendications

1. Système endoscopique (10), comprenant :
une première source de lumière à semi-conducteurs destinée à émettre une première lumière d'éclairage présentant une première longueur d'onde ;
une deuxième source de lumière à semi-conducteurs destinée à émettre une deuxième lumière d'éclairage présentant une deuxième longueur d'onde ; une longueur d'onde isosbestique de l'oxyhémoglobine et de hémoglobine désoxygénée existant entre lesdites première et deuxième longueurs d'onde ;
un dispositif d'imagerie (11) destiné à imager une portion corporelle interne irradiée avec ladite première ou deuxième lumière d'éclairage ;
une section de génération d'image de modèle de vaisseau (60) destinée à produire une image de modèle de vaisseau sur la base d'un signal image obtenu par ledit dispositif d'imagerie (11), incluant
une section de jugement de contraste de vaisseau (61) destinée à juger si oui ou non un contraste de vaisseau entre une zone de vaisseau et une zone autre que ladite zone de vaisseau dans ladite image de modèle de vaisseau coïncide avec une valeur standard, et
une section de réglage de contraste (62, 63, 65) destinée à régler ledit contraste de vaisseau dans le cas où ledit contraste de vaisseau ne coïncide pas avec ladite valeur standard,
**caractérisé en ce que** le système endoscopique comprend en outre une section de génération d'image de saturation en oxygène destinée à produire une image de saturation en oxygène sur la base d'un signal image obtenu par ledit dispositif d'imagerie, le contraste de vaisseau dans un état où le niveau de saturation en oxygène est 100% est la valeur standard, et ladite section de réglage de contraste (62, 63, 65) est configurée pour régler la modification dudit contraste de vaisseau provoquée par une modification d'un niveau de saturation en oxygène du sang dans ladite image de modèle de vaisseau, et
l'intensité lumineuse de ladite première lumière d'éclairage est supérieure à l'intensité lumineuse de ladite deuxième lumière d'éclairage, ou l'intensité lumineuse de ladite deuxième lumière d'éclairage est supérieure à l'intensité lumineuse de ladite première lumière d'éclairage.

2. Système endoscopique (10) selon la revendication 1, dans lequel ladite section de réglage de contraste inclut une section de réglage fin de rapport d'intensité lumineuse (62) destinée au réglage fin d'un rapport d'intensité lumineuse entre lesdites première et deuxième lumières d'éclairage.

3. Système endoscopique (10) selon la revendication 2, dans lequel ladite section de réglage de contraste inclut en outre une section de réglage de ton (65) destinée à régler un ton de ladite image de modèle de vaisseau.

4. Système endoscopique (10) selon la revendication 1, dans lequel ladite section de réglage de contraste inclut une section de conversion de gradation (63) destinée à appliquer un traitement de conversion de gradation à ladite image de modèle de vaisseau.

5. Système endoscopique (10) selon la revendication 2 ou 3, dans lequel ladite section de réglage de contraste inclut en outre une section de conversion de gradation (63) destinée à appliquer un traitement de conversion de gradation à ladite image de modèle de vaisseau.

6. Système endoscopique (10) selon la revendication 5, dans lequel ladite section de réglage fin de rapport d'intensité lumineuse (62) applique un réglage fin audit rapport d'intensité lumineuse entre lesdites première et deuxième lumières d'éclairage, et
ladite section de conversion de gradation (63) applique ledit traitement de conversion de gradation à ladite image de modèle de vaisseau capturée sous irradiation avec lesdites première et deuxième lumières d'éclairage présentant une intensité ayant subi un réglage fin.

7. Système endoscopique (10) selon l'une quelconque des revendications 1 à 6, comprenant en outre un contrôleur de source (35) destiné à contrôler le fonctionnement desdites première et deuxième sources de lumière à semi-conducteurs de telle sorte que lesdites première et deuxième sources de lumière soient appliquées simultanément à ladite portion corporelle interne.

8. Système endoscopique (10) selon l'une quelconque des revendications 1 à 6, comprenant en outre un contrôleur de source (35) destiné à contrôler le fonctionnement desdites première et deuxième sources de lumière à semi-conducteurs de telle sorte que lesdites première et deuxième sources de lumière soient appliquées séquentiellement à ladite portion corporelle interne.

9. Système endoscopique (10) selon l'une quelconque des revendications 1 à 8, pouvant passer d'un mode d'imagerie de modèle de vaisseau, où ladite image de modèle de vaisseau est affichée sur un moniteur, à un mode d'imagerie de saturation en oxygène, où une image de saturation en oxygène est affichée sur ledit moniteur, un niveau de saturation en oxygène du sang étant imagé dans ladite image de saturation en oxygène sur la base dudit signal image.

10. Système endoscopique (10) selon la revendication 9, dans lequel dans ledit mode d'imagerie de saturation en oxygène, il est fait en sorte que ladite intensité lumineuse de ladite première lumière d'éclairage soit supérieure à celle de ladite deuxième lumière d'éclairage, ou il est fait en sorte que ladite intensité lumineuse de ladite deuxième lumière d'éclairage soit supérieure à celle de ladite première lumière d'éclairage, afin d'augmenter la différence de luminosité entre une région hyperoxique présentant ledit niveau élevé de saturation en oxygène et une région hypoxique présentant ledit niveau faible de saturation en oxygène.

11. Système endoscopique (10) selon l'une quelconque des revendications 1 à 10, dans lequel :
D1 ≅D2 est vraie,
où
D1 représente une valeur absolue d'une différence dans un coefficient d'absorption de lumière entre ladite oxyhémoglobine et ladite hémoglobine désoxygénée à ladite première longueur d'onde, et
D2 représente une valeur absolue d'une différence dans ledit coefficient d'absorption de lumière entre ladite oxyhémoglobine et ladite hémoglobine désoxygénée à ladite deuxième longueur d'onde.

12. Système endoscopique (10) selon l'une quelconque des revendications 1 à 10, comprenant en outre :
une troisième source de lumière à semi-conducteurs destinée à émettre une troisième lumière d'éclairage présentant une troisième longueur d'onde vers ladite portion corporelle interne, ladite troisième longueur d'onde étant différente desdites première et deuxième longueurs d'onde, dans lequel
D1 ≅D2+D3 est vraie,
où
D1 représente une valeur absolue d'une différence dans un coefficient d'absorption de lumière entre ladite oxyhémoglobine et ladite hémoglobine désoxygénée à ladite première longueur d'onde ;
D2 représente une valeur absolue d'une différence dans ledit coefficient d'absorption de lumière entre ladite oxyhémoglobine et ladite hémoglobine désoxygénée à ladite deuxième longueur d'onde, et
D3 représente une valeur absolue d'une différence dans ledit coefficient d'absorption de lumière entre ladite oxyhémoglobine et ladite hémoglobine désoxygénée à ladite troisième longueur d'onde.

13. Dispositif processeur (12) d'un système endoscopique (10) incluant un endoscope électronique (11) destiné à imager une portion corporelle interne subissant l'irradiation d'une première lumière d'éclairage présentant une première longueur d'onde et d'une deuxième lumière d'éclairage présentant une deuxième longueur d'onde, une longueur d'onde isosbestique d'oxyhémoglobine et d'hémoglobine désoxygénée existant entre lesdites première et deuxième longueurs d'onde, ledit dispositif processeur (12) comprenant :
une section de génération d'image de modèle de vaisseau (60) destinée à produire une image de modèle de vaisseau sur la base d'un signal image obtenu par ledit endoscope électronique (11), incluant
une section de jugement de contraste de vaisseau (61) destinée à juger si oui ou non un contraste de vaisseau entre une zone de vaisseau et une zone autre que ladite zone de vaisseau dans ladite image de modèle de vaisseau coïncide avec une valeur standard, et
une section de réglage de contraste (62, 63, 65) destinée à régler ledit contraste de vaisseau dans le cas où ledit contraste de vaisseau ne coïncide pas avec ladite valeur standard,
**caractérisé en ce que** le système endoscopique comprend en outre une section de génération d'image de saturation en oxygène destinée à produire une image de saturation en oxygène sur la base d'un signal image obtenu par ledit dispositif d'imagerie, le contraste de vaisseau dans un état où le niveau de saturation en oxygène est 100% est la valeur standard, et ladite section de réglage de contraste (62, 63, 65) est configurée pour modifier ledit contraste de vaisseau provoqué par une modification d'un niveau de saturation en oxygène du sang dans ladite image de modèle de vaisseau, et
l'intensité lumineuse de ladite première lumière d'éclairage est supérieure à l'intensité lumineuse de ladite deuxième lumière d'éclairage, ou l'intensité lumineuse de ladite deuxième lumière d'éclairage est supérieure à l'intensité lumineuse de ladite première lumière d'éclairage.
